# EUROPEAN PATENT APPLICATION

(11) **EP 4 397 240 A1**
(43) Date of publication of application: **10.07.2024**
(21) Application number: 23150330.1
(22) Date of filing: 04.01.2023
(51) Int. Cl.: A61B 5/1455

(54) **OPTICAL SENSOR FOR MEDICAL APPLICATIONS AND SPECIFIC USE OF AN OPTICAL SENSOR**

(71) Applicant: Universität Zürich, 8006 Zürich (CH)
(72) Inventor: KLEISER, Stefan, D-79639 Grenzach-Wyhlen (DE); KALYANOV, Alexander, CH-5507 Mellingen (CH); NÜSSLI, Anais, CH-9500 Will SG (CH); OSTOJIC, Daniel, CH-8046 Zürich (CH)
(74) Representative: Mertzlufft-Paufler, Cornelius

(57) **Abstract**

For improving the accuracy of oxygen saturation measurements in human tissue, an optical sensor (1) is proposed, which features a particular optical measurement arrangement (2) comprising at least one light source (3) and at least one accompanying optical detector (6). The arrangement (2) is distinguished in that it comprises a shielding (7) for shielding light from the at least one optical detector (6). The shielding (7) comprises an active positioning means (11) that can actively position the shielding (7) relative to the at least one light source (3). Thereby at least one, preferably several, source-detector-separation(s) (13) between the at least one light source (3) and the at least one optical detector (6) of the arrangement (2) can be accurately defined, even when the respective light source (3) and/or the respective detector (6) is/are slightly misplaced within the arrangement (2) due to placement tolerances. This approach thus delivers an automatic definition of important optical properties of the arrangement (2), which are relevant to calibration measurements performed with the arrangement (2)

## Description

The present disclosure relates to the field of optical near infrared spectroscopy (NIRS) but can also be employed in other optical measurements. In detail, the disclosure concerns an optical sensor for medical applications, in particular designed for measuring physiological parameters in human or animal tissue. The sensor comprises an optical measurement arrangement with at least one light source arranged in a package or housing and at least one accompanying, preferably functionally connected to each other by a control circuit, optical detector. The at least one optical detector is intended and/or configured to receive light emitted by the at least one light source, after this measurement light has travelled through a portion of the tissue by random scattering processes.

The present disclosure further concerns a specific use of such an optical sensor.

Near-Infrared Spectroscopy (NIRS) is a special spectrophotometric method, which determines concentration of chromophores (for example oxy- and deoxyhemoglobin) in human tissue and, derived from these concentrations, tissue oxygen saturation (StO2). This is accomplished by illuminating the tissue at one or more spots with multispectral sources and measuring the received light intensity at one or more distant points. For this purpose, state-of-the-art oximeters use photodiodes as detectors and various LEDs with different emission wavelengths as light sources.

Optical NIRS sensors are nowadays widely used in clinical monitoring of patients. The accuracy and reliability of the optical measurements performed with such sensors in such applications is highly critical to the well-being of the patient.

It is therefore an object of the present invention to provide an optical sensor as introduced in the beginning which offers improved reliability and/or measurement accuracy. An additional goal of the invention is to simplify the manufacturing and assembly of such a sensor.

In accordance with the present invention, an optical sensor is provided according to claim 1, which solves the aforementioned problems at least partially. In particular, the invention proposes an optical sensor as introduced at the beginning, which is characterized in that the optical measurement arrangement is at least partially covered by a light shielding featuring at least one detector aperture which limits angles, at least in one direction, at which an active area of the at least one optical detector can receive light. Furthermore, it is proposed that the light shielding is self-aligned to the package by a positioning means. Accordingly, the positioning means can be in a direct mechanical contact with the package housing the at least one light source.

As is well known in the art, a light path can be inverted simply by exchanging the position of light source and detector. In such a case, the light shielding may be self-aligned to a package or housing accommodating said at least one optical detector. In other words, in this alternative, which is considered technically equivalent to the invention, the optical sensor (as introduced in the beginning) may be characterized in that the optical measurement arrangement is at least partially covered by a light shielding featuring at least one source aperture which limits angles, at least in one direction, at which the at least one light source can emit light, and the light shielding is self-aligned to the at least one detector, in particular to a housing of said detector, by a positioning means.

The invention thus proposes to align the light shielding directly to the package of the at least one light source. This approach is different from performing such an alignment to structures formed on a PCB. In the latter case, which is often used in the prior art, the placement tolerances of the light sources on the PCB will affect the accuracy of the alignment between the light shielding and the light source. This problem is thus prevented by the invention.

Most preferably, the positioning means can be an active positioning means. Such an active positioning means can provide/produce a positioning force that results in an active alignment of the light shielding w.r.t. the at least one light source.

In other words, thanks to the (in particular active) positioning means, the light shielding can (in particular actively, e.g., by means of a spring action) self-align itself relative to the package accommodating the at least one light source into a final assembly position.

The technical advantage of this approach is that due to the self-alignment/self-aligning through self-positioning of the light shielding relative to the package, the respective position of the at least one detector aperture provided by the light shielding relative to the at least one light source (i.e., in particular, relative to a set of light sources) arranged in the package or at least to the package itself can be accurately and automatically controlled. This greatly simplifies the assembly of the apertures and the total optical measurement arrangement. In addition, the approach proposed by the invention reduces tolerances between the at least one light source - that can be very accurately positioned with respect to the package - and the effective active area of the optical detector or between the active area of the at least one detector and said at least one light source (depending on the design of the light shielding), such that the measurement accuracy is improved.

Another advantage is that the light shielding can be assembled after completion of a pre-assembly of the measurement arrangement on a printed circuit board; holding the light shielding in place during the pre-assembly is thus not necessary. This simplifies the assembly.

More importantly, such an approach is particularly helpful when the optical measurement arrangement is intended for an optical calibration measurement, in which optical coupling efficiencies relevant to optical measurements performed with the at least one detector, are determined (by the optical sensor itself). This is because in such calibration measurements, the effective source-detector-separation (SDS) can be relatively short, and hence any variation of the effective SDS should be minimized, such that relative measurement errors are kept as small as possible.

Preferably, the positioning means can be formed as an integral part of the light shielding.

As already stated, the positioning means may also provide an actuating/positioning force for the self-positioning and/or self-alignment of the light shielding relative to the package. The positioning means may thus not only be a simple alignment structure, but it may actively move the light shielding into a final self-aligned assembly position on the package.

The at least one detector aperture can preferably be formed as an integral part of the light shielding.

According to a preferred embodiment, the light shielding may feature two opposing detector apertures which are symmetrically arranged with respect to a center of said at least one light source. In such a design, the at least one light source (for example located in the center of the measurement arrangement) can be used in conjunction with at least two opposing optical detectors, located below the respective detector aperture, for performing a highly accurate optical calibration measurement.

Alternatively or additionally, in particular if the light shielding is self-aligned to the at least one optical detector, the light shielding may feature two opposing source apertures which are symmetrically arranged with respect to a center of said at least one optical detector. In such a design, the at least one optical detector (for example located in the center of the measurement arrangement) can be used in conjunction with at least two opposing light sources, located below the respective source aperture, for performing a highly accurate optical calibration measurement.

The package or housing accommodating the at least one light source / a set of light sources can provide an optical shielding which prevents direct optical crosstalk between the at least one light source / the light sources and the at least one optical detector. In other words, the package can form an optical barrier between the light sources and the respective optical detector. Alternatively or additionally, such an optical barrier can be formed by the light shielding.

The package may be, in particular, a ceramic package, which can provide excellent optical barrier properties. A combination of both is also possible.

The detector aperture of the light shielding may cover at least part of the active area (i.e., the area of the detector that is sensitive to light) of the at least one optical detector and thus define a sub-area of the active area that can receive light. It is to be understood that the source aperture can be defined within the package housing the light sources, for example. In this case, the relative position of the at least one source aperture to the at least one detector aperture will be defined as soon as the light shielding is self-aligned to the package. The use of a separate source aperture can be highly beneficial because in this case, some misplacement of the respective light source within the package may be tolerated as long as the light source is not covered too much. Depending on the application, some loss of emitted light, due to the coverage, may be acceptable, because the benefit of the source aperture is the improved measurement accuracy.

Alternatively, the source aperture may be integral to the light shielding (the light shielding may thus form the source aperture). This makes the sensor easier to assemble and saves costs.

The optical measurement arrangement, i.e., the at least one light source, in particular said set of light sources, arranged in the package and the at least one optical detector, can be mounted on a common printed circuit board (PCB), which may be designed as a flex-PCB. Thanks to the invention, placement tolerances of the package and the detector - within a certain scope - are rendered irrelevant since the detector aperture will be automatically positioned relative to the package and thus the SDS will be defined mainly by tolerances of the light shielding.

Each of the light sources arranged in the package can emit light around a respective characteristic center wavelength. The package can provide, for example, multiple different measurement wavelengths which are emitted by one of the respective light sources.

The at least one optical detector may comprise multiple receivers that each may be sensitive to all wavelengths or to selected wavelength ranges (e.g. by using appropriate optical filters, which may cover all or parts of the active area of the respective optical detector).

Both the at least one light source and the at least one optical detector of the measurement arrangement can be arranged such that they emit/receive light through a lower contact surface of the optical sensor that is brought into skin contact during an optical measurement.

The optical sensor presented so far can be further elaborated and implemented in various ways, which is described in the sub-claims and in the following:
For example, one embodiment suggests that the positioning means provide(s) a clamping force which clamps the light shielding, preferably onto the package and/or onto the at least one optical detector (in particular onto a package/housing accommodating said at least one detector).

For example, the positioning means may comprise (in particular it may be formed by) at least one spring element. In this case, it is preferred that the at least one spring element is integrally formed as part of the light shielding. The respective spring element can be formed as an elastic lip or an elastic arm, for example. An opposing force, interacting with a positioning force produced by said spring element, may be provided by another portion of the light shielding (for example a delimiter element), and this portion does not necessarily have to form a spring element.

According to a preferred embodiment, the light shielding features two opposing spring elements, for example in the form of two opposing elastically deformable lips or two opposing elastically deformable arms. Moreover, in a final aligned position of the light shielding (= final assembly position), the two opposing spring elements may clamp the package housing the at least one light source and/or the at least one optical detector (in particular a package/housing accommodating said at least one detector) from two opposing sides.

According to another embodiment, which may also be combined with the embodiment featuring two opposing spring elements, the light shielding may feature one spring element and one opposing delimiter element. The latter may provide an opposing force, interacting with the positioning force produced by said spring element. In such a case, these two opposing elements (spring element and opposing delimiter element) may clamp the package from two opposing sides.

For more accurately defining the position of the at least one light source and thus the location of emission of photons from the at least one light source, at least one source aperture may be provided as part of the measurement arrangement which limits angles, at least in one direction, at which the respective light source arranged in the package can emit light. This at least one source aperture may be part of the light shielding or a separate component of the measurement arrangement, in particular integrated into the package holding the at least one ligh source. Advantageously, in such a case, at least one effective source-detector-separation (SDS) between the at least one source aperture and the at least one detector aperture, in particular a respective detector aperture offered by the light shielding, may be defined by the self-aligned light shielding. This approach guarantees a highly precise definition of the SDS, which is important for optical calibration measurements.

We note here that the respective distance between the respective light source(s) and the optical detector(s) varies depending on the placement accuracy of these optoelectronic components on the PCB on which they are mounted. However, due to the use of the self-aligning light shielding, the distance between the respective source aperture(s) and detector aperture(s) (which ultimately defines the average distance that photons will travel from the respective light source to the respective detector and thus the effective SDS) will be unaffected by variations of the placement of the components on the PCB, as long as the detector aperture does not lie outside of the active area in direction of the relevant SDS.

Preferably, the respective detector aperture provided by the light shielding covers a distance in a first direction on the active area of the at least one optical detector that is significantly larger (e.g., 2x or 5x larger) than the placement accuracy of that detector along that first direction. As a result, a variation of the position of the optical detector along the longitudinal axis within the placement accuracy will not change the effective SDS. This is particularly helpful, when using a self-aligning optical shielding as proposed herein in a linear measurement arrangement, in which all optoelectronic components of the arrangement are arranged on a common longitudinal axis; in such a case, only variations of the placement of the components along this longitudinal axis (i.e., in the direction of the relevant SDS) must be compensated for an accurate measurement and this can be effectively achieved with the invention.

The at least one detector aperture provided by the light shielding may reduce an angular range of incoming light rays that the active area of the optical detector located beneath the respective detector aperture can detect. On the other hand, the aforementioned at least one source aperture (which may be part of the light shielding) may reduce an angular range of outgoing light rays that the respective light source, located beneath the respective source aperture, can emit.

Furthermore, the at least one detector aperture may cover two opposing sub-areas, respectively, located at opposing edges of an active area of the respective optical detector located below the respective detector aperture. In this case, the light shielding will cover two opposing edges and adjacent parts of the active area, in particular such that only a central portion of the active area can receive light. This design has the advantage that the width of the active area (in direction from edge to opposing edge) is independent of the placement accuracy. In case the at least one light source offers an extended active area, from which light is emitted, the respective source aperture may cover two opposing edges and adjacent parts of the active area of the light source, in particular such that only a central portion of the active area can emit light.

The angular range at which the active area of the optical detector can detect rays may be understood here as the field of view of the respective detector. Hence each detector aperture, in particular each respective portion of the respective detector aperture, of the light shielding can limit a respective field of view of a detector located below that aperture / aperture portion.

The intentional covering of sub-areas/portions of the active area by the optical shielding thus reduces the active area of the detector that is usable during an optical measurement performed by the optical sensor. In other words, the optical shielding can show a significant overlap, at least in one direction (preferably along a longitudinal axis of the measurement arrangement as described in more detail below), with the active area of the respective optical detector. The benefit is that the size of the active area that can receive light and, in particular, its location relative to the source aperture or light source will remain constant even when the position of the detector below the aperture is not perfectly controlled, which is often the case due to placement tolerances when mounting/reflow-soldering the detector onto a PCB of the optical sensor. A misplacement of the respective chip inside a chip housing will thus be less relevant (at least in the longitudinal direction) since the optical shielding determines the exact place and size of the active (sub-)area of the respective chip that can receive light, regardless of tolerances in the placement of the chip inside the housing.

The at least one source aperture mentioned before may be arranged inside of the package; in particular, it may be formed by the package, which houses the at least one light source. Alternatively, the at least one source aperture may be integrally formed as part of the light shielding.

Moreover, the light shielding may feature a window in which the package (holding the at least one light source) is arranged and through which the at least one light source (housed in the package) can emit light, in particular after the light has passed a respective source aperture. Alternatively, the at least one optical detector may be arranged in said window. In this case, the at least one detector will receive the light through said window.

According to a preferred embodiment, said window may be delimited on two opposing sides by a (respective) spring element, respectively. These two spring elements can thus form the positioning means (aligning the light shielding to the at least one light source or the at least one optical detector).

Another embodiment suggests that the light shielding forms the at least one source aperture (in particular as an integral part of the light shielding) and/or covers at least part of the light sources.

In addition, the light shielding may provide an optical barrier preventing direct optical crosstalk between the at least one light source and the at least one optical detector. Preferably, said optical barrier can be formed as an integral part of the light shielding, for example as a bent flap. The flap may even be soldered to a PCB on which the optical measurement arrangement is mounted. Thereby, tunnelling of light below the flap is effectively prevented.

The at least one source aperture can be formed as a slit aperture, in particular running along a transversal axis. This has the advantage that all light sources of the package can effectively have the same longitudinal coordinate.

Likewise, (in particular each of) the at least one detector aperture provided by the light shielding can be formed as a slit aperture, and this slit aperture may be running along said mentioned transversal axis.

Hence, the slit apertures forming the source aperture and each of the respective one of the at least one detector aperture may be arranged in parallel, respectively, i.e., they may be oriented along the same transversal axis.

One advantageous embodiment proposes that the light shielding offers two detector apertures each designed as a slit aperture and differing (from each other) in their respective slit width. These two detector apertures may be adjoint or separate from each other. In such a case, it is preferable if the two detector apertures are aligned along a common transversal axis.

Moreover, it is advantageous if the two detector apertures cover active areas, respectively, of two separate optical detectors arranged in a common housing. Such a design allows to fine-tune and optimize the respective active area of the respective optical detector arranged in the common housing that can receive light, which is highly beneficial for optimizing the sensitivity of the optical measurement performed with the respective detector. This is particularly true if the two separate optical detectors arranged in the common housing are configured to measure different measurement wavelengths (either by using appropriate optical filtering or time-multiplexing during the measurement), because in this case, each slit aperture can be optimized for use with one of the two different measurement wavelengths. For example, if one of the two different measurement wavelengths is more strongly absorbed in the tissue, this may be compensated by using a larger slit width of the corresponding detector aperture.

The two detector apertures each designed as a slit aperture can be separated by a thin separating bridge, which is blocking light; or the two slit areas of the two detector apertures may be merged into one open aperture. Seen from a different perspective, in such an embodiment the light shielding can offer a slit aperture featuring two neighbouring regions (along the transversal axis), which each region offering a different slit width. For example, the slit width may be smaller in a first region of the slit aperture than in a second region of the slit aperture. The slit width can be understood here as the width of the slit along a longitudinal axis, which is perpendicular to the transversal axis.

The individual widths of the slit apertures can thus vary depending on the optical detector underneath the respective slit aperture and depending on the field of view/amount of light required for that optical detector, which can depend on the source-detector-separation in which this particular optical detector is used in an optical measurement performed by the sensor. This approach is highly useful for maximizing the sensitivity of the individual optical measurements that can be performed with an individual optical detector of the sensor. For example, the longer the effective SDS and/or the stronger the respective measurement wavelength is absorbed in the tissue, the larger the slit width can be chosen to allow a meaningful compromise between spatial resolution (which is favoured by a slim slit aperture) and SNR (which is generally favoured by a broad slit aperture and hence a large active area that can receive photons).

In particular when the light shielding is self-aligned to the at least one optical detector, the light shielding may offer two source apertures, preferably each designed as a slit aperture. In such a case, it is preferable if the two source apertures are both running in the direction of a common transversal axis. For example, the two source apertures may be located to the left and to the right of the at least one optical detector.

It is to be noted here, that the light shielding can feature further optical apertures not designed as a slit. For example, when using an optical detector of the optical measurement arrangement for ambient light sensing, the light shielding can feature a corresponding rectangular aperture matching the full or a part of the active area of that detector used for ambient light sensing.

According to another preferred embodiment, the light shielding forms a Faraday shield which provides electromagnetic shielding for the at least one optical detector. Preferably, the light shielding may be formed as a bent sheet of metal, in particular with the at least one detector aperture and/or the at least one source aperture being cut out of the sheet; or, for example, from a plastic material featuring electrical conductors (e.g., in the form of embedded particles or in the form of a conductive coating applied to the plastic material) which render the plastic material electrically conductive. Such embodiments can all result in an effective Faraday shield. It is understood that the Faraday shield may be electrically grounded, in particular to a ground potential provided by a PCB on which the optical measurement arrangement is assembled.

Following the 2nd alternative of using a plastic material, the light shielding may be 3d-printed from a suitable polymer featuring electrically conductive particles or it may be injection moulded, to name just two possible approaches for fabrication. The two mentioned alternatives may also be combined: for example, the light shielding may be obtained from a bent and pre-cut metal shield, and injection moulded plastic pieces may be added for additional optical shielding, for example to form an additional optical barrier as mentioned above. A suitable metal coating that can enhance the electromagnetic-shielding properties but also simplify a soldering attachment of the optical shielding to a PCB is Zinc.

To greatly improve the robustness of the overall assembly and/or for providing electrical grounding of the light shielding (in particular its Faraday shield), one embodiment suggests that the light shielding features soldering feet which are soldered to a printed-circuit-board (PCB). This PCB may electrically contact the light sources and/or the at least one optical detector. Moreover, the soldering feet may be formed as bent flaps from a metal sheet forming the optical shieling. Each soldering feet may also offer a flat contact surface that can slide over a respective contact pad of the PCB during the self-alignment of the optical shielding. Finally, for simplifying the soldering attachment of the light shielding to the PCB, the light shielding can feature a soldering enhancing coating. The soldering feet can be designed as elastic elements which can provide a flexible mechanical contact to a contact pad of the PCB to which the light shielding is to be soldered.

According to another preferred embodiment, the flaps forming the soldering feet may each be bent from a respective sidewall of the optical shielding which covers a side facet of the at least one optical detector (and thus provide em-shielding to these side facets).

Preferably, a distance between two opposing soldering feet of the light shielding may be chosen such that there always remains at gap between the side facets of the at least one optical detector and the sidewalls of the optical shielding, taking placement tolerances of the at least one optical detector into account. Through this approach and as a result of the self-positioning/self-alignment of the light shielding provided by the active positioning means, the movement of the light shielding will be unimpeded by the at least one optical detector (no matter where exactly the optical detector is located within the placement accuracy).

The advantage of using soldering feet is that the solder connection of the light shielding to the PCB will define the z-height of the at least one detector aperture above the plane of the PCB. When the at least one optical detector is soldered onto the same PCB, the z-distance between the respective detector aperture and the active area at the top of the respective optical detector can thus be accurately controlled, which is important for accurately defining/limiting the field-of-view of that detector. Alternatively, the light shielding can be contacted to the PCB with contacting means such as conductive spring elements.

It is further noted that optical filters may be arranged on top of the respective active area of the respective optical detector comprised in the measurement arrangement. In this case, the respective optical filter (which may be implemented as a thin layer of silicone with embedded colorant with a specific optical absorption band, for example) will be located in between an inner side of the optical shielding and the active area of the respective detector.

According to one preferred embodiment, the optical measurement arrangement comprises at least two opposing optical detectors (preferably two opposing pairs of separate optical detectors arranged in a common housing, respectively) arranged on a longitudinal axis, and the package housing the at least one light source is also arranged on the longitudinal axis in between the two opposing optical detectors. In such an arrangement, it is beneficial if the light shielding features at least two opposing slit apertures, preferably with differing (i.e., different and/or varying) slit widths, which are positioned over the respective optical detector and thus define at least two (preferably differing) source-detector-separations along the longitudinal axis between the at least one light source and the respective optical detector. Such a linear optical measurement arrangement according to the invention is ideally suited for performing multiple optical calibration measurements rapidly and with high accuracy.

Another embodiment suggests that the package comprises at least four lights sources. These light sources may be aligned on a common transversal axis. At least two of the light sources, preferably all four light sources, may be emitting a different wavelength spectrum (e.g., centered about different center wavelengths), respectively. This design allows a multi-wavelength calibration measurement using the at least one optical detector of the arrangement.

Yet another design suggests that, to the left and to the right of the package along a longitudinal axis, two optical detectors may be arranged in a common housing, respectively, and that each active area of the respective optical detector is covered by a respective detector aperture, preferably in the form of a slit aperture. Here again, it may be of advantage depending on the measurement approach for improving the sensitivity of the measurement, if the slit widths of the detector apertures covering the detectors of two different housings differ from each other. Moreover, for the same reason it may be beneficial if at least one of the slit widths of the detector apertures covering the detectors arranged in the same housing differs from another one of the slit widths.

A highly preferred embodiment proposes that the sensor comprises two optical measurement arrangements (which may each be designed as detailed before), each comprising a respective at least one light source, in particular a respective set of light sources, arranged in a respective package, at least one accompanying optical detector, preferably and at least one source aperture. In such a sensor-design, each of the two measurement arrangements may be covered by a respective light shielding featuring at least one detector aperture and being self-aligned to the respective package, as has been previously described. Moreover, the two optical measurement arrangements can be arranged on a common longitudinal axis (to form a linear measurement arrangement) and each detector/source aperture may be designed as a slit aperture oriented along a transversal axis common to both optical measurement arrangements.

As already mentioned, each of the two measurement arrangements can have features as previously explained. The advantage of such a sensor layout is that each optical measurement arrangement can be used for performing optical calibration measurements characterizing the respective optical detector and/or light source used in the measurement and, afterwards, accurate optical measurements, taking the results of the calibration measurements into account, can be performed using (in particular alternately) a light source comprised in one (a first one) of the two optical measurement arrangements and an optical detector comprised in the other (second) optical measurement arrangement (and vice versa).

Finally, the invention suggests to use an optical sensor as described above and/or according to one of the claims directed towards an optical sensor in the following way for achieving the objective mentioned at the beginning: First, the sensor performs at least one optical calibration measurement to determine optical coupling efficiencies and/or correction factors using the at least one light source and the at least one optical detector comprised in the measurement arrangement of the optical sensor. We note that the at least one optical calibration measurement is based on at least one source-detector-separation defined by the optical shielding. It is evident, that the invention thus suggests that the optical sensor, in particular a controller of said optical sensor, may be configured to perform the steps just explained with respect to said use of the sensor.

In other words, the optical sensor first determines the coupling efficiencies and/or correction factors by performing an optical calibration measurement in tissue using the measurement arrangement and the accurate at least one SDS defined by the self-aligned optical shielding. Secondly, the optical sensor can then perform various optical measurements (typically ranging deeper into the tissue than the optical calibration measurement) using further light sources not comprised in the measurement arrangement covered by the light shielding but using a respective optical detector that is covered by a detector aperture provided by the light shielding. If the optical sensor does not move significantly (relative to the skin) during these different measurements, highly accurate measurements of the physiological parameters can be performed with the sensor.

Thus, in particular, a set of light sources housed in the package may be a first set and the optical sensor may feature at least one second set of light sources located on a longitudinal axis at a larger distance from the at least one optical detector (comprised in the measurement arrangement) than the first set. In this case, the optical sensor can measure physiological parameters in human tissue using the at least one second set and the at least one optical detector comprised in the measurement arrangement and considering the optical coupling efficiencies and/or correction factors determined in the optical calibration measurement.

This approach thus proposes to benefit from the accurate definition of relevant source-detector-separations provided by the light shielding according to the invention for performing highly accurate calibration measurements, which can then form the basis for following optical measurements of physiological parameters in human tissue, all using a sensor according to the invention.

Some examples of the present invention will now be described in more detail, although the present invention is not limited to these examples: for those skilled in the art, it is obvious that further examples of the present invention may be obtained by combining features of one or more of the patent claims with each other and/or with one or more features of an example as described or illustrated herein.

With reference to the accompanying drawings, where features with corresponding technical function are referenced with same numerals even when these features differ in shape or design:
- Fig. 1: is a perspective view of an optical sensor according to the invention, featuring three separate optical measurement arrangements,
- Fig. 2: shows a top view on the left outermost optical measurement arrangement of the sensor of Figure 1,
- Fig. 3: shows a top view on the optical measurement arrangement in the center of the sensor of Figure 1,
- Fig. 4: provides a schematic illustration of the lower contact side of the optical sensor of Figure 1,
- Fig. 5 and 6: show details of a light shielding according to the invention, which is part of the optical measurement arrangement of Figure 2,
- Fig. 7 and 8: show details of another light shielding according to the invention, which is part of the optical measurement arrangement of Figure 3,
- Fig. 9: provides a slightly inclined side view on the optical measurement arrangement of Figure 3,
- Fig. 10: provides a top view on the light shielding visible also in Figures 5 and 2 and illustrates two optical detectors positioned below the right detector aperture of the light shielding,
- Fig. 11: shows details of another light shielding according to the invention,
- Fig. 12: shows details of yet another light shielding according to the invention, and
- Fig. 13: shows another possible embodiment of a light shielding 7 according to the invention.

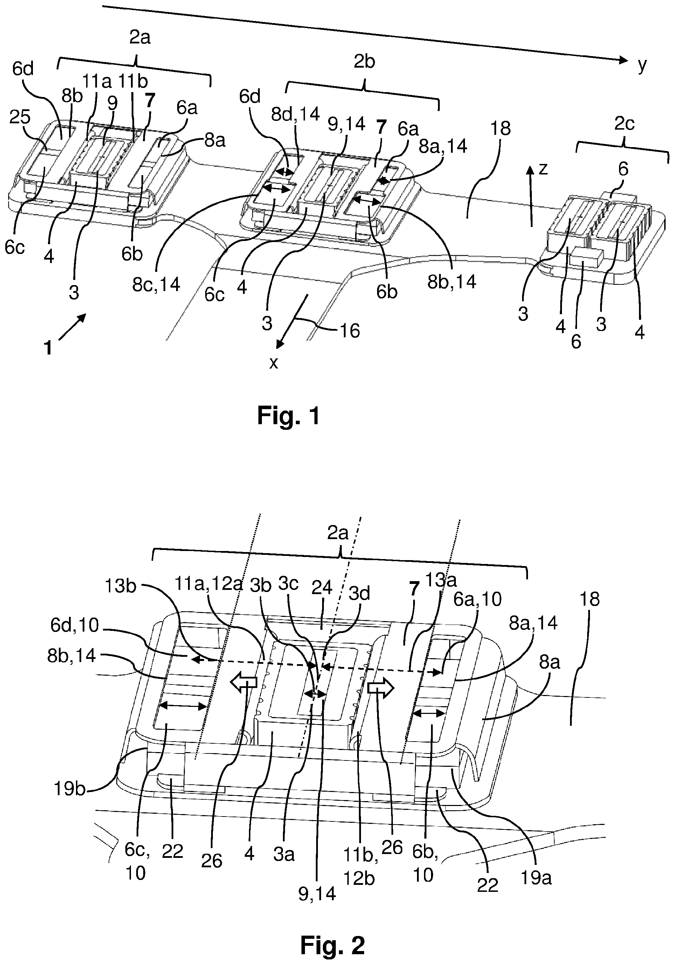

Figure 1 shows an example of an optical sensor 1 according to the invention, which is designed as an optical near-infrared-spectroscopy (NIRS)-sensor. Such sensors are widely used in medical applications for live monitoring of blood oxygenation levels in the brain. In other words, the sensor 1 is capable of measuring blood oxygenation as a physiological parameter in e.g., human tissue, namely in deep lying brain layers.

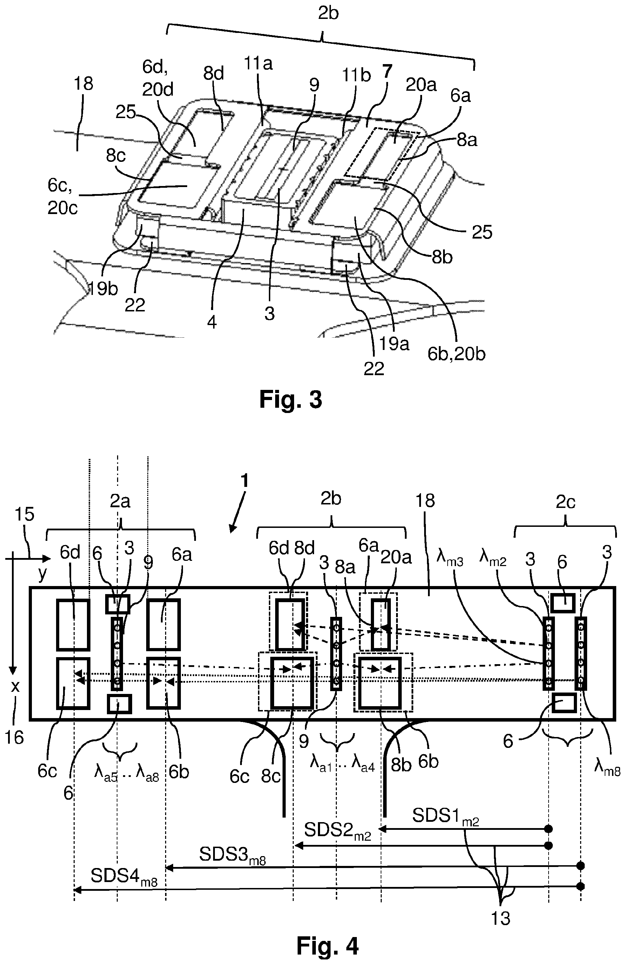

As visible in Figure 4, the sensor 1 comprises three separate optical measurement arrangements 2a, 2b, 2c. Each optical measurement arrangement 2 features at least one light source 3 (namely multiple LEDs) and at least one accompanying optical detector 6, namely at least one respective photodiode. These optoelectronic components are mounted on and electrically contacted by a common printed circuit board (PCB) 18, which may be designed as a flex-PCB. Accordingly, the lower contact surface (here facing the reader) of the optical sensor 1, which is brought into skin contact during a measurement and through which light is emitted and received by the sensor 1, can be bent, such that the sensor 1 can be attached to the curved skull of a patient.

The left outermost optical measurement arrangement 2a, shown in more detail in Figure 2, features a total of four different LEDs (light sources 3a, 3b, 3c, 3d) forming a set of light sources 3, with each LED emitting a distinct and different wavelength ([λa1 .. Xa4] - cf. Figure 4). The four LEDs are housed in a common ceramic package 4, which offers a slit aperture 14 (running along the transversal x-axis 16 of the sensor 1) that forms a source aperture 9. The source aperture 9 defines the exact location in y-direction (cf. Figure 1), at which light from the four LEDs can be emitted (along the z-axis - cf. Figure 1) into brain tissue, when the sensor 1 is placed on the skull of the patient. Moreover, the source aperture 9 limits angles in the y-direction, at which the respective LED arranged in the package 4 can emit light into the tissue. In the example of Figure 2, the source aperture 9 is arranged inside the package 4; however, a technically equivalent implementation would be a separate source aperture 9 that covers the package 4. Indeed, the source aperture 9 may be formed by the light shielding 7.

As visible in Figure 2, the optical measurement arrangement 2a is covered by a light shielding 7 that is formed as a bent metal sheet. As the light shielding 7 is thus electrically conductive and soldered to the PCB 18 using the illustrated soldering feet 22, it can provide an efficient electromagnetic shielding for the optical detectors 6 comprised in the arrangement 2b. The soldering feet 22 are formed as bent flaps and as part of the metal sheet forming the optical shieling 7 and each offer a flat contact surface that can slide over a respective contact pad 5 of the PCB 18 during a self-alignment of the optical shielding 7.

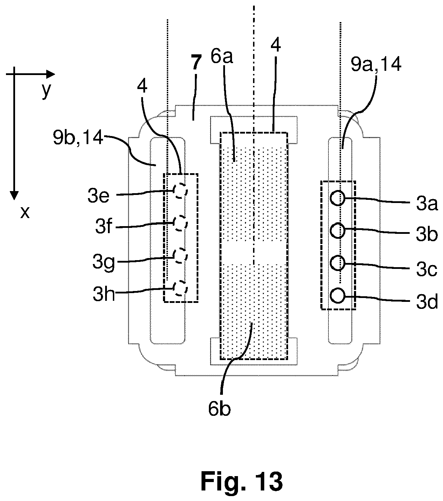

The two dotted lines and the dot-dash-line in between make it easy to recognize in Figure 2 that the light shielding 7 features two opposing detector apertures 8a and 8b, which are symmetrically arranged with respect to a center of said at least one light source 3. In other words, the respective y-distances (cf. Fig. 1) between the respective edge (dotted lines) of the detector aperture 8a/8b and the center line (dot-dash-line), on which the light sources 3 accommodated in the package 4 are arranged, are the same. The symmetrical arrangement is also highlighted by two dotted lines and a dot-dash-line in Figure 4. In fact, both optical measurement arrangements 2a and 2b in Figure 4 (as well as in the example of Figure 1) show such a symmetric arrangement of two opposing detector apertures 8a, 8b. In the example of Figure 13, where the light shielding 7 is self-aligned to the package 4 holding the two centrally arranged optical detectors 6a and 6b, the light shielding 7 offers two opposing source apertures 9a and 9b, which are symmetrically arranged with respect to a center of said at least one detector 6a, and 6b (as visible from the two dotted lines and the dot-dash-line). Also note that the respective slit widths of the two source apertures 9a, 9b differ in Figure 13.

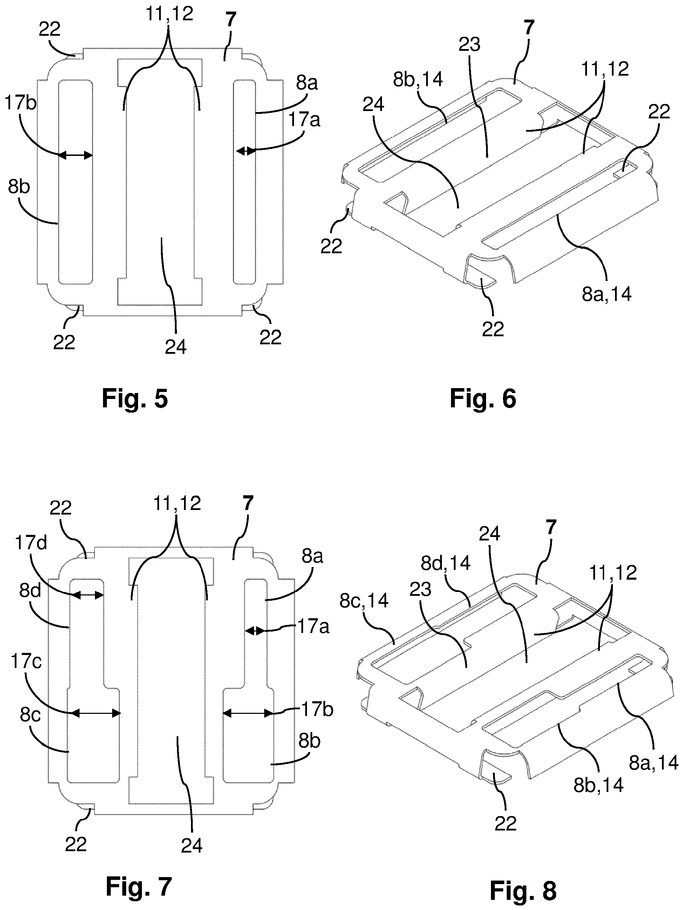

The light shielding 7 shown in Figure 2 features two detector apertures 8a, 8b in the form of slit apertures 14a, 14b with a constant respective slit width 17a/17b (cf. Figure 5). Each of these apertures 8a, 8b can thus limit angles in the y-direction, at which an active area 10 of the respective detector 6 (covered by the light shielding 7) can receive light. This is important for performing meaningful NIRS-measurements, which requires accurately defined effective source-detector-separations (SDS) 13.

As visible in Figure 2 (and in Figures 5 and 6), the slit apertures 14a, 14b are also running along the transversal x-direction 16 of the optical sensor 1, similar to the source aperture 9. In Figure 5, it is notable that the respective slit widths 17a, 17b of the right aperture 8a and the left aperture 8b differ from each other. Such a design can optimize the relative sensitivity of the optical measurements performed at different wavelengths, because different wavelength dependent optical attenuations and differing quantum efficiencies of the detectors 6 for different wavelengths can be considered. In addition, the detectors may be used for different purposes and for light coming from different directions (ambient light, light for calibration, light for the actual measurement etc.). Accordingly, the aperture may have different shapes and may or may not be adjoint. One of the apertures may e.g. be a pin hole, while others form a slit of variable width.

The light shielding 7 of Figure 2 also comprises an active positioning means 11 in the form of two opposing spring elements 12a, 12b. As can be seen, the package 4 holding the set of light sources 3 is placed in a window 24 of the light shielding 7 (cf. Figure 6). Accordingly, the spring elements 12a, 12b, which are integral parts of the light shielding 7 and formed as elastically deformable lips, lie flat against the vertical sidewalls of the package 4. As each spring element 12a, 12b is slightly deformed, each spring element 12a, 12b provides a restoring force that acts as an actuating force 26 which actively aligns the light shielding 7 to the package 7. In other words, thanks to the active positioning means 11, the light shielding 7 can self-align to the package 4. The positioning means 11 also provide a clamping force which holds the light shielding 7 in place, relative to the package 4.

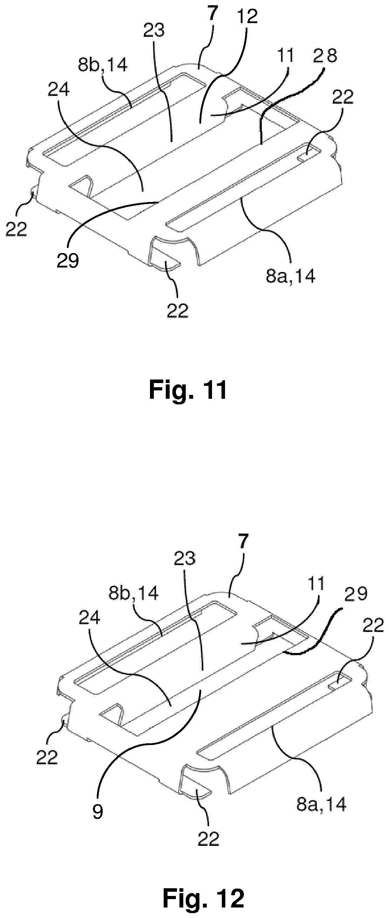

An alternative is shown in figure 11, where one spring element 11 and an opposing delimiter element 28 align the light shielding 7.

As a result, the relative positions/distances of the two detector apertures 8a, 8b of the light shielding 7 are accurately defined w.r.t. the source aperture 9. This relative positioning is important, because the relative locations of the detector apertures 8a, 8b w.r.t. the source aperture 9 define respective effective source-detector-separations (SDS) 13, which are illustrated in Figure 4 by dashed arrows. Accurate control of the respective SDS 13 is important for performing accurate optical calibration measurements with the optical measurement arrangement 2a.

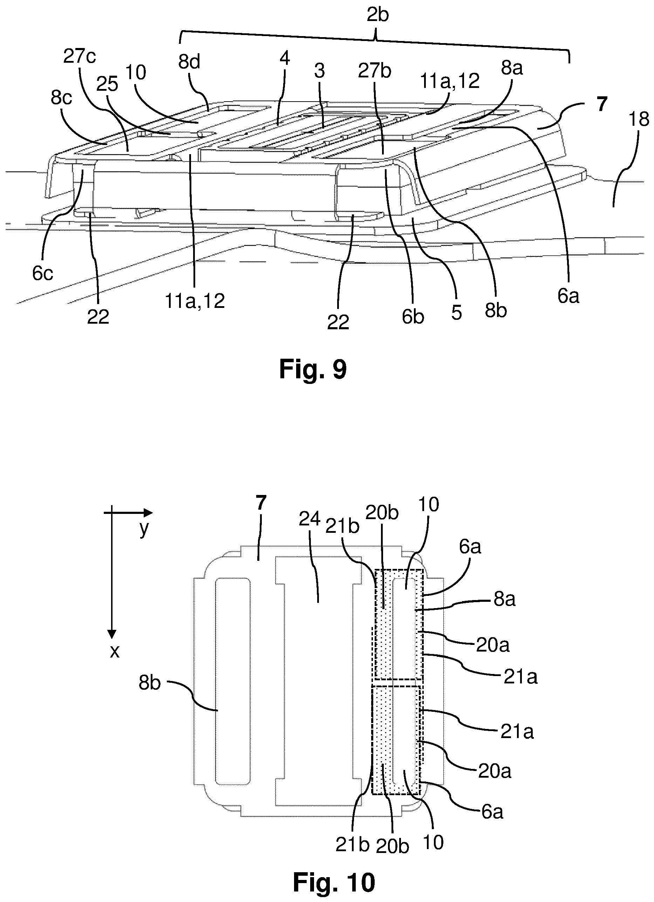

In addition, placement inaccuracies of the respective optical detector 6 beneath the shielding 7, as a result of an assembly process on the PCB 18 (e.g., mounting of the photodiodes by reflow soldering), do not affect the resulting SDS 13. This may be understood with a look onto Figure 10, which illustrates two photodiodes (dashed boxes) as optical detectors 6a, 6b located on the PCB 18 and below the light shielding 7. As can be seen, the respective detector aperture 8a, 8b covers two opposing sub-areas 20a, 20b, respectively, which are located at opposing edges 21a, 21b of the active area 10 of the respective optical detector 6a, 6b which is located below the respective detector aperture 8a, 8b. Hence, some misalignment of the respective photodiode in the y-direction can be tolerated and this will not affect the size of the part of the active area 10, which can receive photons through the respective detector aperture 8a/8b, and, even more important, its relative location w.r.t. to the source aperture 9 (cf. Figure 2).

As may be understood from the perspective view of Figure 9, the respective detector aperture 8 formed by the light shielding 7 will reduce the angular range of incoming light rays that the respective active area 10 of the optical detector 6 beneath the respective detector aperture 8 can detect. This angular range is directly related to the slit width 17 of the respective aperture 8.

According to the design of the light shielding of Figure 2, the two opposing optical detectors 6a, 6d for example, which are arranged on the longitudinal y-axis 15, are approximately symmetrically arranged w.r.t. the light sources 3 arranged in the package 4; however, as the slit widths 17a, 17b and locations of the slit apertures 14a, 14b vary, two different source-detector-separations 13a, 13b are specified by the light shielding 7, as illustrated by the dashes arrows in Figure 2.

With reference to Figure 4, the invention thus suggests that the sensor 1 performs multiple optical calibration measurements to determine (for multiple wavelengths) respective optical coupling efficiencies and/or correction factors using the light sources 3 and the optical detectors 6 comprised in the optical measurement arrangement 2a and/or 2b. In these optical calibration measurements, several source-detector-separation (SDS) 13 as defined by the respective optical shielding 7 are employed (i.e., the respective values for the SDS as specified by the respective shielding 7 are considered during computing of the optical coupling efficiencies and/or correction factors). As a result, these optical calibration measurements will be highly accurate, because the respective SDS 13 is accurately defined by the light shielding 7 and basically independent of placement tolerances of the optical detectors 7 or the package 4 on the PCB 18. Note that the SDS does not need to be measured in direction of the y-axes and may in fact run under an angle thereto (cf. Figure 4).

As can be seen in Figure 1 and 4, the optical sensor 1 not only features two first sets of light sources 3 (namely the light sources 3 comprised in the optical measurement arrangement 2a and 2b) but also a second set of light sources 3 (providing a total of eight different measurement wavelength [λm1 .. Am8]) as part of the right outermost optical measurement arrangement 2c. All these light sources 3 are arranged on the longitudinal axis 15 of the optical sensor 1 (cf. Figure 1). However, the light sources 3 of the arrangement 2c are located at a larger distance from the optical detectors 6 of the arrangement 2b than the first set of light sources 3 of the arrangement 2b. The same is true with respect to the optical detectors 6 of the arrangement 2a and its set of light sources 3. As can be seen in Figure 4, optical measurements with large SDS 13 can thus be performed using the light sources 3 of the right outermost arrangement 2c (i.e., with said second set of light sources 3) and using optical detectors 6 of the arrangements 2a and/or 2b. We note at this point, that a large SDS 13 is tantamount to a large penetration depth of the measurement wavelength λₘᵢ into the tissue, i.e., such measurements are suitable for optically probing deep brain layers.

As a result, the optical sensor 1 can thus accurately measure the blood oxygenation as a physiological parameter in human tissue using the described second set of light sources 3 of the arrangement 2c and using the optical detectors 6 comprised in the measurement arrangements 2a and 2b. In these optical measurement, the optical coupling efficiencies and/or correction factors (determined in the precedent optical calibration measurements) are considered, which greatly improves the measurement accuracy. The use of the optical shielding 7 according to the invention thus results in a higher accuracy of the optical calibration measurements and thereby, ultimately, also in a higher accuracy of the performed optical measurements of said physiological parameter. In total, the accuracy of the oxygen saturation measurements is thus improved, to the benefit of the patient.

Figure 3 shows another embodiment of a light shielding 7 according to the invention, which covers the center optical measurement arrangement 2b that was already shown in Figure 1. It is notable, that the shielding 7 offers four slit apertures 8a, 8b, 8c, 8d which cover the respective active areas 10 of four different optical detectors 6a, 6b, 6c, 6d, each in the form of a respective photodiode. Seen from another perspective, the slit widths 17a and 17d of the detector apertures 8a and 8d covering the detectors 6a and 6d, which are located in two different separate housings 19a, 19b (cf. Figure 3) differ from each other, as visible in Figure 7. In addition, the slit widths 17a and 17b of the detector apertures 8a and 8b covering the detectors 6a and 6b which are arranged in the same housing 19a also differ from each other. As a result, a total of four different regions of variable size are defined w.r.t. the respective active region 10 of the respective optical detector 6 by the light shielding 7. Hence, it is possible to measure four different wavelengths with optimized sensitivity using the four detector apertures 8a, 8b, 8c, and 8d. As there are a two optical measurement arrangements 2a, 2b, for each of the eight measurement wavelengths a corresponding optical calibration measurement can be performed, using the auxiliary wavelength Aai.

In summary, an optical sensor 1 is proposed which features a particular optical measurement arrangement 2 comprising at least one light source 3 and at least one accompanying optical detector 6. The arrangement 2 is distinguished in that it comprises a shielding 7 for shielding light from the at least one optical detector 6. The shielding 7 possesses an active positioning means 11 that can actively position the shielding 7 relative to the at least one light source 3. Thereby at least one, preferably several, source-detector-separation(s) 13 between the at least one light source 3 and the at least one optical detector 6 of the arrangement 2 can be accurately defined, even when the respective light source 3 and/or the respective detector 6 is/are slightly misplaced within the arrangement 2 due to placement tolerances. This approach thus delivers an automatic definition of important optical properties of the arrangement 2, which are relevant to calibration measurements performed with the arrangement 2.

Figures 11 and 12 show each a respective embodiment of a light shielding 7 according to the invention: In the example of Figure 11, the light shielding 7 features one spring element 12 that forms an active positioning means 11 that can produce a positioning force, and a delimiter element 28 (formed as an edge 29) opposing said spring element 12. In the final assembly position of the light shielding 7 (not shown in the figure), both the delimiter element 28 and the spring element 12 rest against the package 4 accommodating the at least one light source 3 of the optical measurement arrangement 2 and these two elements 12, 28 clamp the package 4 from two opposing sides.

In the example of Figure 12, the light shielding 7 features an edge 29 that forms a source aperture 9 for the light source 3 (not shown in the Figure) of the optical measurement arrangement 2 that is covered by the light shielding 7. In this embodiment, this edge 29 does not rest against the package 4 accommodating the at least one light source 3 but instead covers (partly) the at least one light source 3 / said package 4. In other words, the embodiment of Figure 12 is an example of a source aperture 9 that is formed as an integral part of the light shielding 7.

Figure 13 shows an embodiment of a light shielding 7 according to the invention, in which the light shielding features a source aperture 9a (limiting angles, at least in one direction, at which the respective light source 3a / 3b / 3c / 3d can emit light into the tissue), which is self-aligned to a package / housing 4 accommodating two optical detectors 6a, 6b. In this embodiment, the position of said package 4 / said detectors 6a, 6b will thus define the final position of the light shielding 7. The detectors 6a, 6b and the four light sources 3a - 3d (as well as the further four light sources 3e - 3h) may be part of an optical measurement arrangement 2 as detailed before. In this embodiment, placement tolerances of the respective light source 3 shown in Figure 13 will not affect the effective SDS between the respective light source 3 and the respective detector 6. This is because the respective source aperture 9a, 9b is aligned to both detectors 6a, 6b and the locations where the respective light source 3 can emit light into the tissue is thus defined by the light shielding 7, not by the position of the light source 3 (as long as the placement tolerance is not so large that the light source is completely blocked by the light shielding 7; note for example that the left light sources 3e - 3f are partly blocked by the light shielding 7, which will lead to a certain loss of light but can still result in a reasonable measurement of high accuracy).

Compared to the other embodiments, the light path has been inverted in the example of Figure 13 by exchanging the position of the at least one optical detector 6 and the at least one light source 3; however the light shielding 7 could also be aligned to the housing/package 4 accommodating the at least one optical detector 6 without exchanging the positions of light source and detector: for example, when aligning the light shielding 7 to the detectors 6 shown in Figure 2, the source aperture 9 would then be positioned above the light sources 3 in the center of the arrangement 2.

### List of reference numerals

- 1: optical sensor
- 2: optical measurement arrangement
- 3: light source (e.g., a LED or a laser diode)
- 4: package (housing several 3 or one / several 6)
- 5: contact pad (of 18)
- 6: optical detector (e.g., a photodiode)
- 7: light shielding
- 8: detector aperture
- 9: source aperture
- 10: active area (of 6)
- 11: positioning means
- 12: spring element
- 13: source-detector-separation (SDS)
- 14: slit aperture
- 15: longitudinal axis
- 16: transversal axis
- 17: slit width (of 14)
- 18: printed-circuit-board
- 19: housing (for 6)
- 20: sub-area (of 10)
- 21: edge (of 10)
- 22: soldering feet
- 23: optical barrier
- 24: window (for receiving 4 or 6)
- 25: separation (between 6)
- 26: actuating force (provided by 11/12)
- 27: optical filter layer (e.g., provided in the form of a thin silicone film comprising absorbents than can block certain wavelengths to be suppressed)
- 28: delimiter element
- 29: edge

## Claims

1. **Optical sensor (1)** for medical applications, in particular for measuring physiological parameters in human or animal tissue, the sensor (1) comprising
- an optical measurement arrangement (2) with at least one light source (3) arranged in a package (4) and at least one accompanying optical detector (6),
**characterized in that**
- the optical measurement arrangement (2) is at least partially covered by a light shielding (7), and
- the light shielding (7) features at least one detector aperture (8a, 8b) which limits angles, at least in one direction, at which an active area (10) of the at least one optical detector (6) can receive light, and
the light shielding (7) is self-aligned to the package (4) by a positioning means (11) and/or
- the light shielding (7) features at least one source aperture (9a, 9b) which limits angles, at least in one direction, at which the at least one light source (3) can emit light, and the light shielding (7) is self-aligned to the at least one optical detector (6) by a positioning means (11).

2. Optical sensor (1) according to claim 1, wherein the light shielding (7) features two opposing detector apertures (8a, 8b) which are symmetrically arranged with respect to a center of said at least one light source (3) and/or
- wherein the light shielding (7) features two opposing source apertures (9a, 9b) which are symmetrically arranged with respect to a center of said at least one optical detector (6).

3. Optical sensor (1) according to claim 1 or claim 2, wherein the positioning means (11) is an active positioning means (11), in particular providing a positioning force,
- in particular wherein the positioning means (11) provides a clamping force which clamps the light shielding (7), preferably onto the package (4) and/or onto the at least one optical detector (6), and/or
- wherein the positioning means (11) comprise at least one spring element (12),
- preferably wherein the at least one spring element (12) is integrally formed as part of the light shielding (7) .

4. Optical sensor (1) according to one of the preceding claims, wherein the light shielding (7) features two opposing spring elements (12a, 12b), for example in the form of two opposing elastically deformable lips or one spring element (12a) and one opposing delimiter element (28) .
- in particular such that in a final aligned position of the light shielding (7), the two opposing elements (12a, 12b/ 12, 28) clamp the package (4) housing the at least one light source (3) and/or clamp the at least one optical detector (6) from two opposing sides.

5. Optical sensor (1) according to one of the preceding claims, wherein at least one source aperture (9) limits angles, at least in one direction, at which the respective light source (3) arranged in the package (4) can emit light, preferably wherein the source aperture (9) is integrally formed as part of the light shielding (7), and
- wherein at least one effective source-detector-separation (13) between the at least one source aperture (8) and the at least one detector aperture (8) is defined by the self-aligned light shielding (7).

6. Optical sensor (1) according to one of the preceding claims, wherein the at least one detector aperture (8) reduces an angular range of incoming light rays that the active area (10) of the optical detector (6) beneath the respective detector aperture (8) can detect and/or wherein the at least one source aperture (9) reduces an angular range of outgoing light rays that the respective light source (3), located beneath the respective source aperture (9), can emit and/or
- wherein the at least one detector aperture (8) covers two opposing sub-areas (20a, 20b), respectively, located at opposing edges (21a, 21b) of an active area (10) of the respective optical detector (6) located below the respective detector aperture (8).

7. Optical sensor (1) according to one of the preceding claims, wherein the at least one source aperture (9) is arranged inside of the package (4), in particular formed by the package (4), which houses the at least one light source (3), and/or
- wherein the light shielding (7) features a window (24) in which the package (4) is arranged and through which the light sources (3) can emit light,
- preferably wherein the window (24) is delimited on two opposing sides by the positioning means (11), preferably formed by respective elements (12a, 12b/ 12, 28).

8. Optical sensor (1) according to one of the preceding claims, wherein the light shielding (7) forms the at least one source aperture (8) and/or covers at least part of the light sources (3) and/or
- wherein the light shielding (7) provides an optical barrier (23) preventing direct optical crosstalk between the at least one light source (3) and the at least one optical detector (6), preferably wherein the optical barrier (23) is formed as an integral part of the light shielding (7), for example as a bent flap.

9. Optical sensor (1) according to one of the preceding claims, wherein the at least one source aperture (9) is formed as a slit aperture (14), in particular running along a transversal axis (16), and/or
- wherein, in particular each of, the at least one detector aperture (8) provided by the light shielding (7) is formed as a slit aperture (14), in particular running along the transversal axis (16).

10. Optical sensor (1) according to one of the preceding claims, wherein the light shielding (7) offers two detector apertures (8a, 8b), preferably each designed as a slit aperture (14) and differing in their respective slit width (17),
- preferably wherein the two detector apertures (8a, 8b) are aligned along a common transversal axis (16) and/or respectively cover active areas (10a, 10b) of two separate optical detectors (6a, 6b / 6c, 6d) arranged in a common housing (19) and/or
- wherein the light shielding (7) offers two source apertures (9a, 9b), preferably each designed as a slit aperture (14).

11. Optical sensor (1) according to one of the preceding claims, wherein the light shielding (7) forms a faraday shield which provides electromagnetic shielding for the at least one optical detector (6), and/or
- wherein the light shielding (7) is formed
- as a bent sheet of metal, in particular with the at least one detector aperture (8) being cut out of the sheet, or
- from a plastic material featuring electrical conductors, for example in the form of embedded particles or in the form of a conductive coating, which render the plastic material electrically conductive.

12. Optical sensor (1) according to one of the preceding claims, wherein the light shielding (7) features soldering feet (22) which are soldered to a printed-circuit-board (18) which preferably electrically contacts the light sources (3) and/or the at least one optical detector (6),
- in particular wherein the soldering feet (22) are formed as bent flap from a metal sheet forming the optical shieling (7) and/or each offer a flat contact surface that can slide over a respective contact pad of the printed-circuit-board (18) during the self-alignment of the optical shielding (7), and/or
- wherein the light shielding (7) features a soldering enhancing coating.

13. Optical sensor (1) according to one of the preceding claims, wherein the optical measurement arrangement (2) comprises at least two opposing optical detectors (6a, 6c; 6b, 6d) arranged on a longitudinal axis (15),
- the package (4) housing the at least one light source (3) is arranged on the longitudinal axis (15) in between the two opposing optical detectors (6a, 6b), and
- the light shielding (7) features at least two opposing slit apertures (14a, 14c; 14b, 14d), preferably with different and/or varying slit widths (17a, 17c; 17c, 17d), which are positioned over the respective optical detectors (6a, 6c; 6b, 6d) and thus define at least two, preferably different and or varying, source-detector-separations (13a, 13b) along the longitudinal axis (15) between the at least one light source (3) and the respective optical detector (6a, 6b, 6c, 6d).

14. Optical sensor (1) according to one of the preceding claims, wherein the package (4) comprises at least four lights sources (3), preferably aligned on a common transversal axis (16), and at least two of the four light sources (3) may be emitting a different wavelength spectrum, respectively and/or
- wherein to the left and to the right of the package (4) along a longitudinal axis (15) two optical detectors (6a, 6b; 6c, 6d) are arranged in a common housing (19a, 19b), respectively, and wherein each active area (10) of the respective optical detectors (6a, 6b, 6c, 6d) is covered by a respective detector aperture (8), preferably in the form of a slit aperture (14),
- preferably wherein the slit widths (17) of the detector apertures (8) covering the detectors (6a, 6c) of two different housings (19a, 19b) differ from each other, and/or
- wherein at least one of the slit widths (17) of the detector apertures (8) covering the detectors (6a, 6b / 6c, 6d) arranged in the same housing (19a / 19b) differs from another one.

15. Optical sensor (1) according to one of the preceding claims, wherein the sensor (1) comprises two optical measurement arrangements (2a, 2b) each comprising a respective at least one light source (3), in particular a respective set of light sources (3), arranged in a respective package (4a, 4b), at least one accompanying optical detector (6), preferably and at least one source aperture (9), and
- wherein each measurement arrangement (2a, 2b) is covered by a respective light shielding (7a, 7b) featuring at least one detector aperture (8) and being self-aligned to the respective package (4a, 4b),
- wherein the two optical measurement arrangements (2a, 2b) are arranged on a common longitudinal axis (15) and each aperture (8, 9) is designed as a slit aperture (14) oriented along a transversal axis (16) common to both optical measurement arrangements (2a, 2b).

16. **Use of an optical sensor (1)** according to one of the preceding claims,
- wherein the sensor (1) performs at least one optical calibration measurement to determine optical coupling efficiencies and/or correction factors using the at least one light source (3) and the at least one optical detector (6) comprised in the optical measurement arrangement (2) and
- wherein the at least one optical calibration measurement is based on at least one source-detector-separation defined by the optical shielding (7),
- in particular wherein the set of light sources (3) is a first set and wherein the optical sensor (1) features at least one second set of light sources (3) located on a longitudinal axis (15) at a larger distance from the at least one optical detector (6) than the first set, and wherein the optical sensor (1) measures physiological parameters in human or animal tissue using the at least one second set and the at least one optical detector (6) comprised in the measurement arrangement (2) and taking into account the optical coupling efficiencies and/or correction factors determined in the optical calibration measurement.
